# EUROPEAN PATENT SPECIFICATION

(11) **EP 3 406 724 B1**
(45) Date of publication and mention of the grant of the patent: **14.12.2022**
(21) Application number: 17741627.8
(22) Date of filing: 17.01.2017
(51) Int. Cl.: C12N 15/63, C12N 15/52, C12N 9/02, C12N 9/10, C12N 9/88, C12P 7/40

(54) **RECOMBINANT MUTANT MICROORGANISMS HAVING ACRYLIC ACID PRODUCTIVITY AND METHOD FOR PRODUCING ACRYLIC ACID USING SAME**
REKOMBINANTE MUTANTE MIKROORGANISMEN MIT ACRYLSÄUREPRODUZIERENDER AKTIVITÄT UND VERFAHREN ZUR HERSTELLUNG VON ACRYLSÄURE DAMIT
MICRO-ORGANISMES MUTANTS RECOMBINANTS PRÉSENTANT UNE PRODUCTIVITÉ D'ACIDE ACRYLIQUE ET PROCÉDÉ DE PRODUCTION D'ACIDE ACRYLIQUE LES UTILISANT

(30) Priority: 18.01.2016 KR 20160005941
(43) Date of publication of application: 28.11.2018
(73) Proprietor: Hanwha Chemical Corporation, Seoul 04541 (KR); Korea Advanced Institute of Science and Technology, Daejeon 34141 (KR)
(72) Inventor: LEE, Sang Yup, Daejeon 34049 (KR); KO, Yoo Sung, Daejeon 34141 (KR); SONG, Chan Woo, Daejeon 34141 (KR); KIM, Je Woong, Daejeon 34141 (KR)
(74) Representative: Regimbeau
(86) International application number: PCT/KR2017/000558
(87) International publication number: WO 2017/126861

(56) References cited:
- WO-A1-2013/044076
- WO-A1-2013/044076
- KR-A- 20150 130 830
- US-A1- 2012 149 077
- US-A1- 2014 099 676
- US-A1- 2016 010 124
- GLORIA HERRMANN ET AL: "Two beta-alanyl-CoA:ammonia lyases in Clostridium propionicum", FEBS JOURNAL, vol. 272, no. 3, 1 February 2005 (2005-02-01), pages 813-821, XP055324319, GB ISSN: 1742-464X, DOI: 10.1111/j.1742-4658.2004.04518.x -& DATABASE EMBL [Online] 18 May 2004 (2004-05-18), "Clostridium propionicum acl2 gene, act gene and bar gene", XP002791096, retrieved from EBI accession no. EM_STD:AJ715482 Database accession no. AJ715482
- NICOLE LINDENKAMP ET AL: "A propionate CoA-transferase of Ralstonia eutropha H16 with broad substrate specificity catalyzing the CoA thioester formation of various carboxylic acids", APPLIED MICROBIOLOGY AND BIOTECHNOLOGY, vol. 97, no. 17, 19 December 2012 (2012-12-19), pages 7699-7709, XP055241903, DE ISSN: 0175-7598, DOI: 10.1007/s00253-012-4624-9
- JONATHAN D. TODD ET AL: "The Ruegeria pomeroyi acuI Gene Has a Role in DMSP Catabolism and Resembles yhdH of E. coli and Other Bacteria in Conferring Resistance to Acrylate", PLOS ONE, vol. 7, no. 4, 26 April 2012 (2012-04-26), page e35947, XP55586434, DOI: 10.1371/journal.pone.0035947
- CHAN WOO SONG ET AL: "Metabolic engineering of Escherichia coli for the production of 3-aminopropionic acid", METABOLIC ENGINEERING, vol. 30, 7 June 2015 (2015-06-07), pages 121-129, XP55586351, US ISSN: 1096-7176, DOI: 10.1016/j.ymben.2015.05.005

## Description

### [Technical Field]

The present invention relates to a recombinant modified microorganism producing acrylic acid, and to a method for preparing acrylic acid using the same. More specifically, the present invention relates to a modified microorganism producing acrylic acid, in which genes encoding an enzyme converting beta-alanine to beta-alanyl-CoA and an enzyme converting beta-alanyl-CoA to acrylyl-CoA are introduced, and to a method for preparing acrylic acid using the same.

### [Background Art]

Acrylic acid is an unsaturated carboxylic acid, and is a chemical material widely used in the production of various industrial products such as plastics, adhesives, coating agents, *etc.* Acrylic acid is mostly produced through oxidation of petrochemical-based propylene. However, the production of acrylic acid through propylene generates a large amount of carbon dioxide during processing, and the necessity for bio-based acrylic acid produced from renewable resources is increasing due to the continuous increase and volatility in the price of propylene.

The direct production of acrylic acid using a modified microorganism has been reported in a dissertation, where it is disclosed that acrylic acid can be produced by utilizing 3-hydroxypropionic acid from glucose in *E. coli* as a precursor and by utilizing 3-HP-CoA dehydratase and CoA hydrolase (Chu HS et al., Metab Eng., 32: 23-29, 2015).

However, the production of acrylic acid using *beta*-alanine has not been reported.

The article (Hermann et al., FEBS Journal, Vol. 272, 2005) describes the pathway of biotransformation of *beta-*alanine to acryloyl-CoA, in the strain *Clostridium propionicum,* but production of acrylic acid is not achieved with this strain.

Although there is a patent directed to a production pathway of acrylic acid using *beta-*alanine, the patent does not clearly specify the enzyme converting *beta*-alanine to *beta*-alanyl-CoA (US 2012/0149077 A1). In addition, there are patents that include a diagram of a metabolic pathway showing the production of acrylic acid using a microorganism (US 7846707 B2 and US 8043841 B2). However, in these patents, the enzyme converting *beta*-alanine to *beta*-alanyl-CoA and the enzyme converting acrylyl-CoA to acrylic acid are not specified; the description regarding the metabolic pathway is not disclosed in the claims; and embodiments demonstrating that acrylic acid is indeed produced are not reported.

Finally, the international application WO 2013/044076 relates to a modified microorganism comprising a biosynthetic pathway that produces *beta*-alanine, then acryloyl-Coa, and further acrylic acid. However, this microorganism produces only traces of acrylic acid, and is not modified for increasing such production.

### [Disclosure]

### [Technical Problem]

The present inventors have made intensive efforts to develop a microorganism producing acrylic acid from a carbon source via a biological pathway. As a result, they have developed enzymes producing acrylic acid under *in vitro* conditions using *beta*-alanine as a substrate. In addition, they also have found that acrylic acid can be produced from *beta*-alanine when using a modified microorganism in which genes encoding an enzyme converting *beta*-alanine to *beta-*alanyl-CoA and an enzyme converting *beta*-alanyl-CoA to acrylyl-CoA are introduced into a modified microorganism that is a *beta*-alanine-producing strain, thereby completing the present invention.

### [Technical Solution]

An objective of the present invention is to provide a modified microorganism producing acrylic acid.

Another objective of the present invention is to provide a method for preparing acrylic acid using the microorganism.

Still another objective of the present invention is to provide a method for preparing acrylic acid from *beta*-alanine under *in vitro* conditions.

### [Advantageous Effects]

Acrylic acid can be prepared from *beta*-alanine by a biological method using the modified microorganism according to the present invention. In addition, the amount of acrylic acid to be produced can be increased through additional enzyme modification of the modified microorganism, and thus the present invention can be effectively used for the industrial production of acrylic acid.

### [Brief Description of the Drawings]

Fig. 1 shows a metabolic pathway for producing acrylic acid from *beta*-alanine.
Fig. 2 is a diagram showing a method for manufacturing a metabolic pathway of a modified microorganism producing acrylic acid from glucose.
Fig. 3 is an image showing that *beta*-alanine coenzyme A transferase is constructed in a *pET-30(a)+* plasmid including a 6-histidine tag for purification.
Fig. 4 is an image showing that *beta*-alanyl-CoA:ammonia lyase is constructed in a *pET-30(a)+* plasmid including a 6-histidine tag for purification.
Fig. 5 is an image showing that propionate coenzyme A transferase is constructed in a *pET-30(a)+* plasmid including a 6-histidine tag for purification.
Fig. 6 is an SDS-PAGE image showing the purification of *beta*-alanine coenzyme A transferase.
Fig. 7 is an SDS-PAGE image showing the purification of *beta*-alanyl-CoA:ammonia lyase.
Fig. 8 is an SDS-PAGE image showing the purification of propionate coenzyme A transferase.
Fig. 9 shows the results of analyzing *beta*-alanyl-CoA prepared using a *beta*-alanine coenzyme A transferase enzyme under *in vitro* conditions.
Fig. 10 shows the results of analyzing acrylic acid prepared using *beta*-alanine coenzyme A transferase and propionate coenzyme A transferase under *in vitro* conditions.
Fig 11 shows a *pTrc99A act* plasmid in which an *act* gene, prepared to express beta-alanine coenzyme A transferase in a microorganism, is inserted.
Fig. 12 shows a *pTrc99A act acl2* plasmid in which *act* and *acl2* genes, prepared to express beta-alanine coenzyme A transferase and *beta*-alanyl-CoA:ammonia lyase in a microorganism, are inserted.
Fig. 13 shows a *pKYS1* plasmid in which *act, acl2, and pct* genes, prepared to express *beta-*alanine coenzyme A transferase, *beta*-alanyl-CoA:ammonia lyase, and propionate coenzyme A transferase in a microorganism, are inserted.

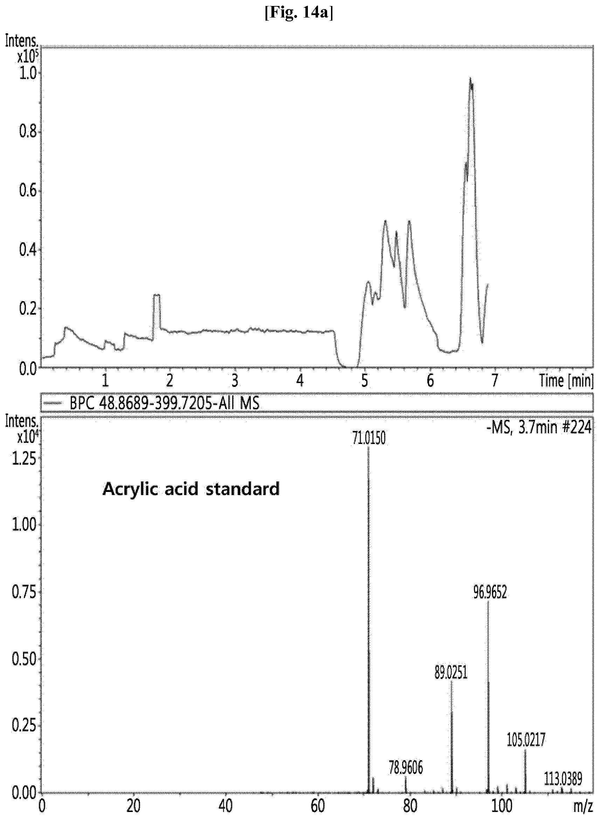

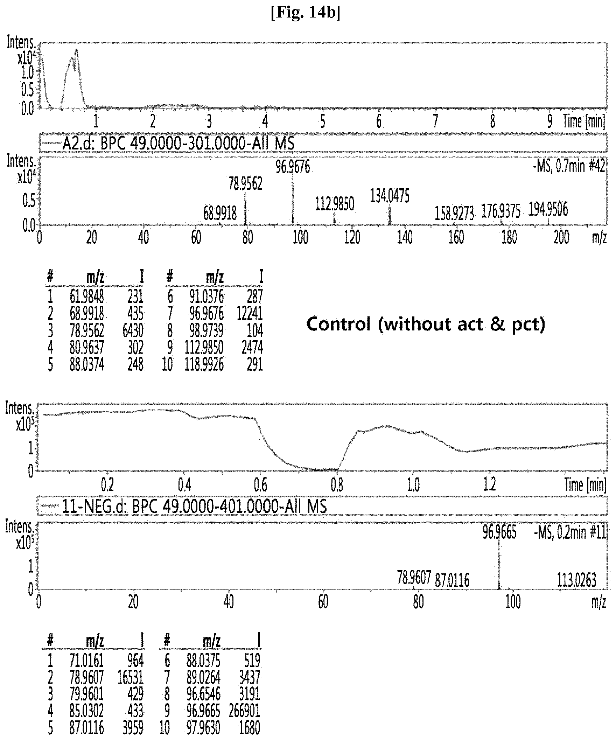

Fig. 14 shows the results of analyzing acrylic acid prepared using modified microorganisms in which the plasmid vectors above are introduced.

### [Best Mode]

Unless defined otherwise, all of the technical and scientific terms used herein have the same meanings as those generally understood by one of ordinary skill in the art to which the present invention pertains. Generally, the nomenclature used herein is well known and commonly employed in the art.

In order to achieve the objectives above, an aspect of the present invention provides a modified microorganism producing acrylic acid, wherein the microorganism is a *beta*-alanine-producing strain, comprising a *act* gene derived from *Clostridium propionicum* encoding a beta-alanine coenzyme A transferase, a *acl2* gene derived from *Clostridium propionicum* encoding a beta-alanyl-CoA:ammonia lyase gene and *apct* gene derived from *Ralstonia eutropha* encoding a propionate coenzyme A transferase; and wherein an *acuI* gene derived from *Escherichia coli* encoding acrylyl-coA reductase is deleted in the microorganism; and wherein the microorganism is *Escherichia* sp.

Additionally, the present invention also provides a method for preparing acrylic acid, comprising: (a) culturing the modified microorganism to produce acrylic acid; and (b) recovering the acrylic acid produced.

The present invention also provides a method for preparing acrylic acid from *beta*-alanine under *in vitro* conditions including preparing acrylic acid, comprising: mixing and reacting beta-alanine coenzyme A transferase encoded by an *act* gene derived from *Clostridium propionicum,* beta-alanyl-CoA:ammonia lyase encoded by an *acl2* gene derived from *Clostridium propionicum* and propionate coenzyme A transferase encoded by *a pct* gene derived from *Ralstonia eutropha* in a reaction solution comprising beta-alanine.

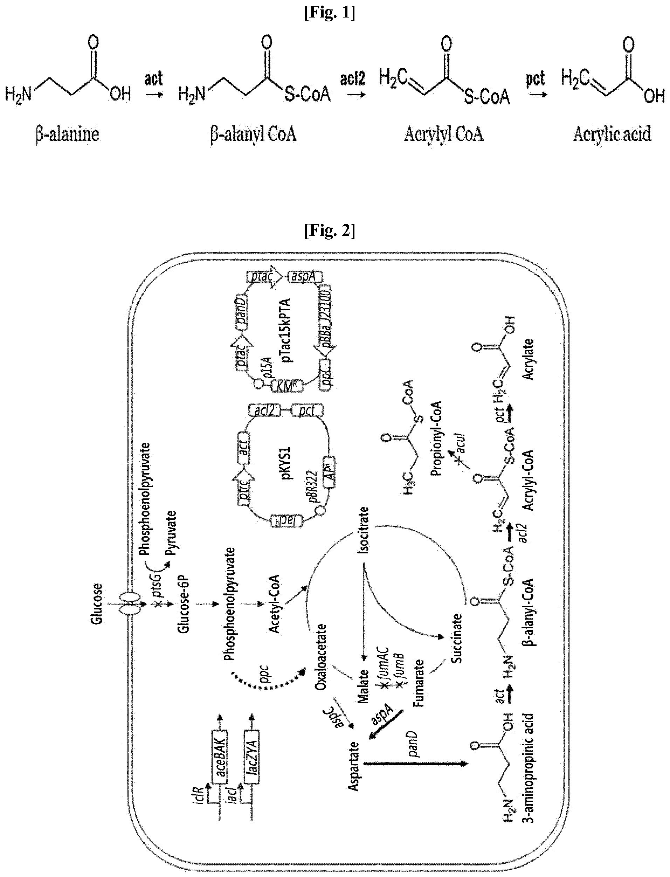

It was confirmed in the present invention that acrylic acid can be produced from *beta-*alanine by using *beta*-alanine coenzyme A transferase and *beta*-alanyl-CoA:ammonia lyase, and based on the above, a system capable of producing acrylic acid from *beta*-alanine by a biological method was established (Fig. 1).

Specifically, in the present invention, an experiment was conducted to investigate whether acrylic acid can be prepared by using a microorganism in which *beta*-alanine coenzyme A transferase and *beta*-alanyl-CoA:ammonia lyase are introduced into a *beta*-alanine-producing strain (Song et al., Metab Eng., 30: 121-129, 2015). As a result, it was confirmed that acrylic acid was produced when using a microorganism into which the enzymes above are introduced (Table 2).

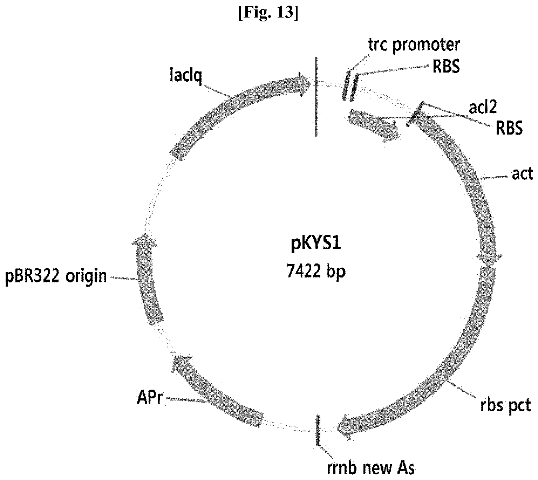

Additionally, in the Examples of the present invention, in order to confirm whether *beta-*alanine is converted to acrylic acid in a microorganism, a *pKYS1* plasmid was constructed in which *act, acl2, and pct* genes encoding *beta*-alanine coenzyme A transferase, *beta*-alanyl-CoA: ammonia lyase, and propionate coenzyme A transferase are cloned (Fig. 13), and then this plasmid was introduced into a *beta*-alanine-producing strain. The modified microorganism was cultured in a medium containing a carbon source, and as a result, it was confirmed that acrylic acid was produced in the microbial medium (Table 2).

Therefore, in an aspect, the present invention relates to a modified microorganism producing acrylic acid, wherein the microorganism is a *beta*-alanine-producing strain, comprising a *act* gene derived from *Clostridium propionicum* encoding a beta-alanine coenzyme A transferase, a *acl2* gene derived from *Clostridium propionicum* encoding a beta-alanyl-CoA:ammonia lyase gene and a *pct* gene derived from *Ralstonia eutropha* encoding a propionate coenzyme A transferase ; and wherein an *acuI* gene derived from *Escherichia coli* encoding acrylyl-coA reductase is deleted in the microorganism; and wherein the microorganism is *Escherichia* sp.

As used herein, the term "acrylic acid" refers to an organic compound having a composition of CH₂=CHOCO₂H, and is also designated as prop-2-enoic acid, *etc.* The modified microorganism of the present invention is one in which 1) *beta*-alanine coenzyme A transferase gene, which is an enzyme converting a substrate *beta*-alanine to *beta*-alanyl coenzyme A, and 2) *beta*-alanyl-CoA:ammonia lyase gene, which is an enzyme converting a substrate *beta*-alanyl coenzyme A to acrylyl coenzyme A, are introduced into a microorganism having an ability to produce *beta*-alanine from a carbon source, and thus the modified microorganism of the present invention has productivity of acrylic acid.

In the present invention, the introduction can be carried out by transformation, and the term "transformation" refers to the introduction of genes into a host cell for the expression of the same. The method for transforming the genes of the present invention into the cells may include any method for introducing the nucleotides into the cells, and it can be performed by selecting an appropriate standard technique known in the art. A method such as electroporation, calcium phosphate co-precipitation, retroviral infection, microinjection, DEAE-dextran, cationic liposome, and thermal shock can be used, but is not limited thereto.

The transformed gene may be inserted into the chromosome of a host cell and located outside the chromosome, as long as it can be expressed in the host cell. In addition, the gene comprises DNA and RNA as a polynucleotide encoding a polypeptide, and any gene that can be introduced and expressed in the host cell can be used without limitation. For example, the gene can be introduced into a host cell in the form of an expression cassette which is a polynucleotide construct, comprising all elements required for self-expression. The expression cassette usually comprises a promoter operably linked to the gene, a transcription termination signal, ribosome binding sites, and a translation termination signal. The expression cassette may be in the form of a self-replicable expression vector. In addition, the gene may be one introduced into a host cell by itself or in the form of a polynucleotide construct and operably linked to the sequences required for expression in the host cell.

As used herein, the term "modified microorganism" refers to a cell transfected with the gene encoding at least one target protein. In the present invention, the microorganism belongs to *Escherichia* sp..

Additionally, the microorganism uses a microorganism that is a *beta*-alanine-producing strain as a host to produce acrylic acid by the introduction of the genes. The *beta*-alanine-producing strain may be, for example, the *CWF4NA pSynPPC7* strain used in the Examples of the present invention (Song et al., Metab Eng., 30: 121-129, 2015), but is not particularly limited thereto as long as the objectives of the present invention can be achieved.

In the present invention, the beta-alanine coenzyme A transferase is an enzyme encoded by an *act* gene derived from *Clostridium propionicum.*

Specifically, the *act* gene derived from *Clostridium propionicum* may include the nucleotide sequence of SEQ ID NO: 1. Additionally, the *act* gene may include a nucleotide having a homology to the nucleotide sequence of SEQ ID NO: 1 of at least 80%, 90%, 95%, 96%, 97%, 98%, or 99%. For example, as long as the gene includes a nucleotide sequence which has such homology and which encodes an enzyme that has an effect corresponding to that of *beta-*alanine coenzyme A transferase, it is apparent that the gene can be included within the scope of the present invention even if it has a nucleotide sequence in which some sequence has been deleted, modified, substituted, or added. As used herein, the term "homology" refers to a percentage of identity between two polynucleotide or polypeptide moieties. Sequence correspondence from one moiety to another can be determined by a known technique in the art. For example, homology may be determined by directly arranging the sequence information (*i.e*., parameters, such as score, identity, similarity, *etc.*) between two polynucleotide molecules or two polypeptide molecules using a computer program (Example: BLAST 2.0) that is easily accessible and capable of arranging sequence information. In addition, homology between polynucleotides may be determined by hybridizing polynucleotides under the condition for forming a stable double strand between the homologous regions and disassembling with a single strand-specific nuclease, followed by size determination of the disassembled fragments.

In the present invention, the *beta*-alanyl-CoA:ammonia lyase is an enzyme encoded by an *acl2* gene derived from *Clostridium propionicum.*

Specifically, the *acl2* gene derived from *Clostridium propionicum* may include the nucleotide sequence of SEQ ID NO: 2. Additionally, the *acl2* gene may include a nucleotide having a homology to the nucleotide sequence of SEQ ID NO: 2 of at least 80%, 90%, 95%, 96%, 97%, 98%, or 99%. For example, as long as the gene includes a nucleotide sequence which has such homology and which encodes an enzyme that has an effect corresponding to that of *beta-*alanyl-CoA: ammonia lyase, it is apparent that the gene can be included within the scope of the present invention even if it has a nucleotide sequence in which some sequence has been deleted, modified, substituted, or added.

In the present invention, the microorganism is a *beta*-alanine-producing strain in which a propionate coenzyme A transferase gene is further introduced. The propionate coenzyme A transferase refers to an enzyme that converts a substrate acrylyl coenzyme A to acrylic acid.

In the present invention, the propionate coenzyme A transferase is an enzyme encoded by *a pct* gene derived from *Ralstonia eutropha.*

Specifically, the *pct* gene derived from *Ralstonia eutropha* may include the nucleotide sequence of SEQ ID NO: 3. Additionally, the *pct* gene may include a nucleotide having a homology to the nucleotide sequence of SEQ ID NO: 3 of at least 80%, 90%, 95%, 96%, 97%, 98%, or 99%. For example, as long as the gene includes a nucleotide sequence which has such homology and which encodes an enzyme that has an effect corresponding to that of propionate coenzyme A transferase, it is apparent that the gene can be included within the scope of the present invention even if it has a nucleotide sequence in which some sequence has been deleted, modified, substituted, or added.

As used herein, the term "vector" refers to a DNA construct including a nucleotide sequence encoding a desired protein, which is operably linked to an appropriate expression regulatory sequence to express the desired protein in a suitable host cell. The regulatory sequence may include a promoter that can initiate transcription, an optional operator sequence for regulating the transcription, a sequence encoding a suitable mRNA ribosome binding site, and a sequence regulating the termination of transcription and translation. After the vector is introduced into the suitable host cell, it may replicate or function independently of the host genome, and may be integrated into the genome itself.

The vector used in the present invention is not particularly limited, as long as it is able to replicate in the host cell, and any vector known in the art may be used. Examples of conventional vectors may include a natural or recombinant plasmid, cosmid, virus, and bacteriophage. For instance, *pWE15, M13, MBL3, MBL4, IXII, ASHII, APII, t10, t11, Charon4A,* and *Charon21A* may be used as a phage vector or cosmid vector; and *pBR* type, *pUC* type, *pBluescriptII* type, *pGEM* type, *pTZ* type, *pCL* type, and *pET* type may be used as a plasmid vector. A vector usable in the present invention is not particularly limited, and any known expression vector may be used. Specifically, *pDZ, pACYC177, pACYC184, pCL, pECCG117, pUC19, pBR322, pAM118,* or *pCC1BAC* vector may be used. More specifically, a *pTrc99A* plasmid capable of expressing a strong gene through a Trc promoter may be used, but is not limited thereto.

In the present invention, the microorganism is a *beta-*alanine-producing strain in which an acrylyl-coA reductase gene is further deleted. The acrylyl-coA reductase refers to an enzyme that converts a substrate acrylyl coenzyme A to propionyl-CoA. In addition, the gene is deleted for the purpose of increasing the production of acrylic acid by blocking the enzyme.

In the present invention, deletion of the gene may include a partial or full deletion of the gene encoding the protein. The partial or full deletion of the gene encoding the protein may be done by introducing a vector for chromosomal insertion in a host cell, thereby substituting the polynucleotide encoding an endogenous target protein on a chromosome with a marker gene or a polynucleotide having a partial deletion of a nucleic acid sequence. For example, a method of deleting the gene by homologous recombination may be used. Additionally, the "partial" deletion may vary depending on the type of polynucleotide, but specifically refers to 1 to 300, more specifically 1 to 100, and further more specifically 1 to 50 nucleotides, but is not particularly limited thereto.

In the present invention, the *acuI* gene derived from *Escherichia coli* may include the nucleotide sequence of SEQ ID NO: 4. Additionally, the *acuI* gene may include a nucleotide having a homology to the nucleotide sequence of SEQ ID NO: 4 of at least 80%, 90%, 95%, 96%, 97%, 98%, or 99%. For example, as long as the gene includes a nucleotide sequence which has such homology and which encodes an enzyme that has an effect corresponding to that of acrylyl-CoA reductase, it is apparent that the gene can be included within the scope of the present invention even if it has a nucleotide sequence in which some sequence has been deleted, modified, substituted, or added.

In the present invention, the microorganism is a *beta*-alanine-producing strain in a medium containing at least one carbon source. As the carbon source contained in the medium, glucose, sucrose, lactose, galactose, maltose, xylose, glycerol, fructose, molasses, starch, cellulose, and sugar cane may be used individually or by mixing; as a nitrogen source, a nitrogen-containing organic compound (for example, peptone, yeast extract, gravy, malt extract, corn steep liquor, soybean flour, and urea), or an inorganic compound (for example, ammonium sulfate, ammonium chloride, ammonium phosphate, ammonium carbonate, and ammonium nitrate) and the like may be used individually or by mixing; and as a phosphorus source, potassium dihydrogen phosphate, dipotassium hydrogen phosphate, a sodium-containing salt corresponding thereto, and the like may be used individually or by mixing. The culture medium may also include essential growth-promoting materials such as other metal salts (for example, magnesium sulfate or iron sulfate), amino acids, and vitamins.

In another aspect, the present invention relates to a method for preparing acrylic acid, comprising: (a) culturing the modified microorganism in a medium to produce acrylic acid; and (b) recovering the acrylic acid produced.

As used herein, the term "culture" refers to cultivation of the microorganism under moderately controlled environmental conditions. The culturing process of the present invention can be carried out according to a suitable culture medium and culture conditions known in the art. Such culturing process can be easily adjusted by one of ordinary skill in the art depending on the strain to be selected. Specifically, the culture may be a batch culture, a continuous culture, or a fed-batch culture, but is not limited thereto.

Chemical compounds such as ammonium hydroxide, potassium hydroxide, ammonia, phosphoric acid, and sulfuric acid may be appropriately added to the incubator during culture to adjust the pH of the culture medium. During incubation, production of bubbles can be inhibited by using an anti-foaming agent such as fatty acid polyglycol ester. Further, in order to maintain the aerobic condition of the incubator, oxygen or an oxygen-containing gas can be added to the incubator, or in order to maintain anaerobic conditions, nitrogen, hydrogen, or carbon dioxide gas may be injected or gas may not be injected. The temperature of the incubation is typically between 27°C to 37°C, specifically 30°C to 35°C, but is not limited thereto. The incubation can be continued until the desired amount of useful material is achieved, and specifically, the incubation period may be 10 hours to 100 hours, but is not limited thereto.

The step of recovering the acrylic acid produced during the culturing step of the present invention can collect desired acrylic acid from a culture medium by using a suitable method known in the art according to the culturing method. For example, the recovering method may be centrifugation, filtration, anion-exchange chromatography, crystallization, HPLC, *etc.,* and the desired acrylic acid may be recovered from a medium or a microorganism by using a suitable method known in the art.

In another Example of the present invention, it was confirmed that acrylic acid was produced under *in vitro* conditions by mixing and reacting *beta*-alanine coenzyme A transferase encoded by an *act* gene derived from *Clostridium propionicum,* beta-alanyl-CoA:ammonia lyase encoded by an *acl2* gene derived from *Clostridium propionicum* and propionate coenzyme A transferase encoded by a *pct* gene derived from *Ralstonia eutropha,* in a reaction solution containing *beta*-alanine.

Therefore, in still another aspect of the present invention, the method for preparing acrylic acid is a method for preparing acrylic acid, comprising: mixing and reacting *beta*-alanine coenzyme A transferase encoded by an *act* gene derived from *Clostridium propionicum,* beta-alanyl-CoA: ammonia lyase encoded by an *acl2* gene derived from *Clostridium propionicum* and propionate coenzyme A transferase encoded by *a pct* gene derived from *Ralstonia eutropha,* in a reaction solution containing *beta*-alanine.

In the present invention, the reaction solution may further contain acetyl-CoA. However, as long as the *beta*-alanine coenzyme A transferase enzyme in the reaction solution can provide CoA to convert *beta*-alanine to beta-alanyl-CoA, the type of reaction solution is not particularly limited.

In the method of the present invention for preparing acrylic acid, propionate coenzyme A transferase may further be mixed, but the method is not limited thereto.

In the present invention, the *beta*-alanine coenzyme A transferase is an enzyme encoded by an *act* gene derived from *Clostridium propionicum.*

In the present invention, the *beta*-alanyl-CoA:ammonia lyase is an enzyme encoded by an *acl2* gene derived from *Clostridium propionicum.*

In the present invention, the propionate coenzyme A transferase is an enzyme encoded by *a pct* gene derived from *Ralstonia eutropha.*

In the present invention, the *beta*-alanine coenzyme A transferase, *beta*-alanyl-CoA:ammonia lyase, and propionate coenzyme A transferase may be separated into whole cell enzymes or partially purified enzymes, but are not limited thereto.

### [Mode for the Invention]

Hereinafter, the present invention will be described in further detail with reference to examples. It will be obvious to one of ordinary skill in the art that these examples are illustrative purposes only and are not to be construed to limit the scope of the present invention.

### Example 1: Confirmation of cloning and activity of beta-alanine coenzyme A transferase

### 1-1: Preparation of pET30a_his_act vector

PCR was carried out with the primers of SEQ ID NOS: 5 and 6 using chromosomal DNA of *Clostridium propionicum* ATCC 25522 as a template. Thereafter, a his-act fragment encoding beta-alanine coenzyme A transferase labeled with a 6-histidine tag at the N-terminus was prepared.
[SEQ ID NO: 5] pET30a-his tagged act (n-term) Fwd: tttaagaaggagatatacatatgAAAAGACCCTTGGAAGGTATTC
[SEQ ID NO: 6] pET30a-his tagged act (n-term) Rev: gcggccgcaagcttgtcgacttaatgatgatgatgatggtgGATGACATTTTTCTCTTCCAG

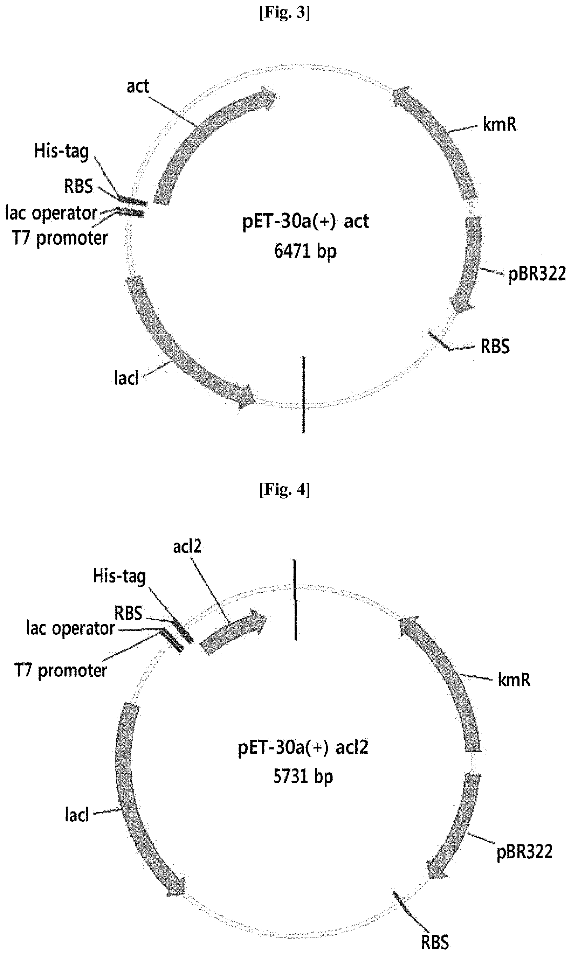

A *pET30a* plasmid (Novagen, USA) capable of expressing a strong gene through the prepared his-act fragment and a T7 promoter were treated with restriction enzymes (*NdeI* and *SalI*)*.* Thereafter, a *pET30a_his_act* expression vector was constructed by ligating the his-act fragment to the *pET30a_*plasmid through the Gibson Assembly reaction (Fig. 3).

### 1-2: Purification of beta-alanine coenzyme A transferase

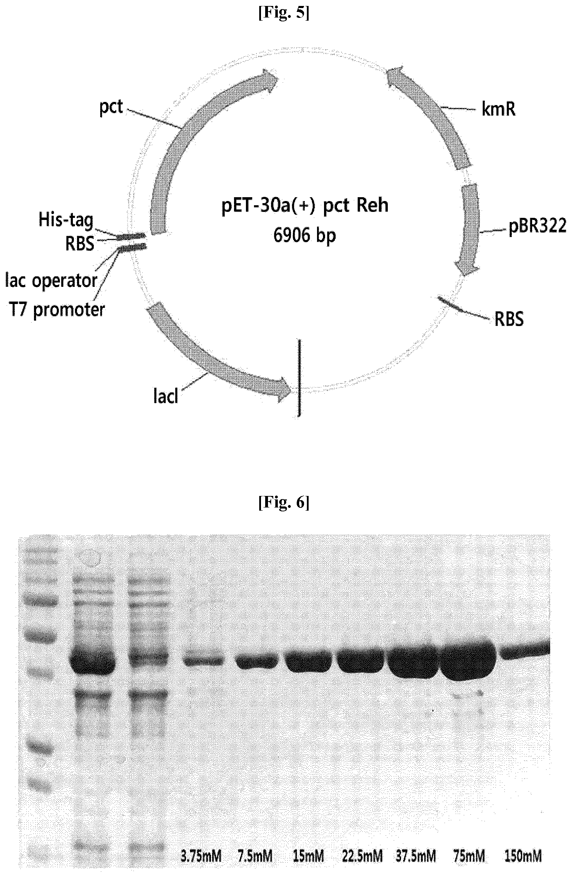

For purification of the beta-alanine coenzyme A transferase, the *pET30a_his_act* plasmid obtained in Example 1-1 was introduced into *E. coli* BL21 (DE3) (F- ompT hsdSB(rB- mB-) gal dcm (DE3), a prophage carrying the T7 RNA polymerase gene) (New England Biolabs, USA). After 12 hours of early incubation wherein the transformed strains were inoculated into a 10 mL LB liquid medium (tryptone (10 g/L), yeast extract (5 g/L), NaCl (10 g/L)) containing kanamycin (25 mg/L) and continuously shaken at 37°C at 20 rpm, the cells were inoculated 1% in 400 mL of the medium described above and incubated at 37°C with constant shaking at 200 rpm. Thereafter, 1 mM IPTG was added to induce *his_act* expression when the optical density (OD) measured at a wavelength of 600 nm reached 0.55 to 0.66 in a spectrophotometer. After 3 hours of expression induction, the culture medium was centrifuged for 10 minutes at 3000 rpm at 4°C in a centrifuge (Hanil Science Industrial, Korea) to isolate microorganisms, and then the supernatant was removed. Thereafter, the isolated microorganisms were mixed in 35 mL of an equilibrium buffer (50 mM Na₃PO₄, 300 mM NaCl, pH 7.0), then were disrupted by way of pulsing for 5 seconds at 30% intensity using a cell sonicator (cell sonicator, Sonics & Materials, Inc., USA) and allowing to stand for 5 seconds for 1.5 hours, and then were centrifuged at 4°C at 13200 rpm for 10 minutes to remove cell debris and obtain a cell lysate. The cell lysate was poured into a filter (0.45 µm), and the his-tagged *beta-*alanine coenzyme A transferase was isolated using Talon resin (Clontech Laboratories, Inc., USA). Isolation of *beta*-alanine coenzyme A transferase attached on the Talon resin was carried out using equilibrium buffers each containing 3.75, 7.5, 15, 22.5, 37.5, 75, and 150 mM imidazole. Thereafter, the samples, in which the whole cell lysate and the protein solution obtained with each concentration of imidazole, were mixed with a 5× Laemmli sample buffer solution (LPS Solution, Korea) and separated using 12% SDS-PAGE and stained with a Coomassie brilliant blue R250 (Bio-Rad, USA) solution (Fig. 6).

As a result, it was confirmed that the *beta*-alanine coenzyme A transferase purified with 15 mM imidazole had the highest purity. In addition, the buffer was exchanged by flowing a 50 mM potassium phosphate buffer (pH 7.5) using an Amicon Ultra Centrifugal Filter (Merck Millipore, Germany). The purified *beta*-alanine coenzyme A transferase was mixed with glycerol at a volume ratio of 1:1, and then stored at -80°C.

### Example 1-3. Analysis of activity of beta-alanine coenzyme A transferase enzyme

For the analysis of enzyme activity, *beta*-alanine (10 mM) and acetyl-CoA (1 mM) were dissolved in 50 mM of a potassium phosphate buffer (pH 7.5), and 15 µg of the purified *beta-*alanine coenzyme A transferase was added. The reaction was carried out at 37°C for 5 hours. An OASIS HLB SPE cartridge (Waters, USA) was used in order to isolate only the coenzyme A derivatives from the reaction mixture, and the following protocol was used.

In the first cartridge, methanol (1 mL) was poured, a 0.15% TCA solution (2 mL) was poured, and then the reaction mixture was poured. Thereafter, the 0.15% TCA solution (1 mL) was poured therein once again.

Lastly, 1 mL of a solution in which methanol and ammonia water are mixed in a volume ratio of 99:1 was poured, and then purified CoA derivatives were obtained. The solvent was then removed using a vacuum centrifuge, and the sample was stored at -24°C. The sample was dissolved in distilled water, and the analysis of the CoA derivatives was conducted by HPLC-MS (Mass spectrometers: LC/MSD VL, Agilent, USA, HPLC: Agilent, USA) or HPLC-MS/MS analysis (Mass spectrometers: API3200QTRAP, SCIEX, USA, HPLC: Shimadzu, Japan).

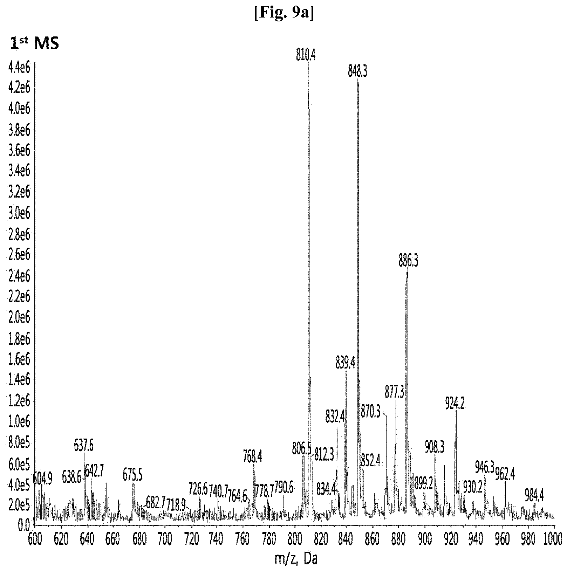

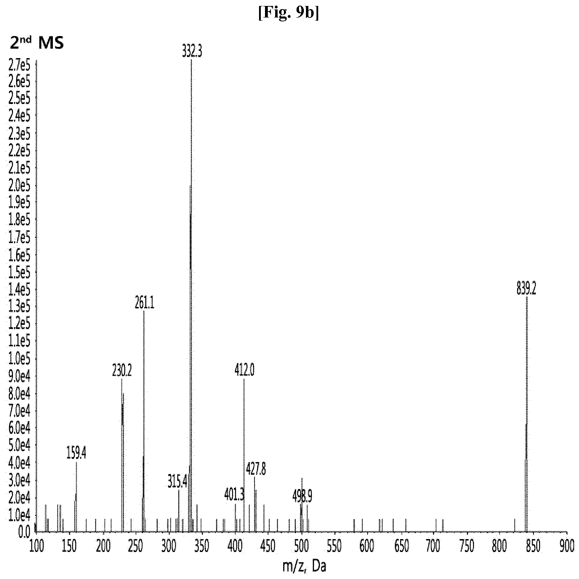

Primary MS analysis of the reaction mixture was carried out using *beta*-alanine as a substrate, and as a result, a peak was confirmed at 837.7, which is similar to the expected m/z value of 838.613 of GABA coenzyme A. This peak was then fragmented with secondary MS and the analysis was carried out. As a result, peaks were confirmed at 230.2, 332.2, and 427.8, which are similar to the expected peaks *m*/*z* = 230, 332, and 428, respectively (Fig. 9).

From these results, it was confirmed that *beta*-alanine coenzyme A transferase successfully converted beta-alanine to *beta*-alanyl-CoA according to the present invention.

### Example 2: Confirmation of cloning and activity of beta-alanyl-CoA:ammonia lyase

### 2-1. Preparation of pET30a_his_acl2 vector

PCR was carried out with the primers of SEQ ID NOS: 7 and 8 using chromosomal DNA of *Clostridium propionicum* ATCC 25522 as a template. Thereafter, a his-act fragment encoding beta-alanyl-CoA:ammonia lyase labeled with a 6-histidine tag at the N-terminus was prepared.
[SEQ ID NO: 7] pET30a-his tagged acl2 (n-term) Fwd: tttaagaaggagatatacatatgcaccatcatcatcatcatGTAGGTAAAAAGGTTGTACATC
[SEQ ID NO: 8] pET30a-his tagged acl2 (n-term) Rev: cggagctcgaattcggatccTTATTGAGGGTGCTTTGC

A *pET30a* plasmid (Novagen, USA) capable of expressing a strong gene through the prepared his-act fragment and a T7 promoter were treated with restriction enzymes (*NdeI* and *SalI*)*.* Thereafter, a *pET30a_his_act* was constructed by ligating the his-acl2 fragment to the *pET30a_* plasmid through the Gibson Assembly reaction (Fig. 4).

### 2-2. Purification of beta-alanyl-CoA:ammonia lyase

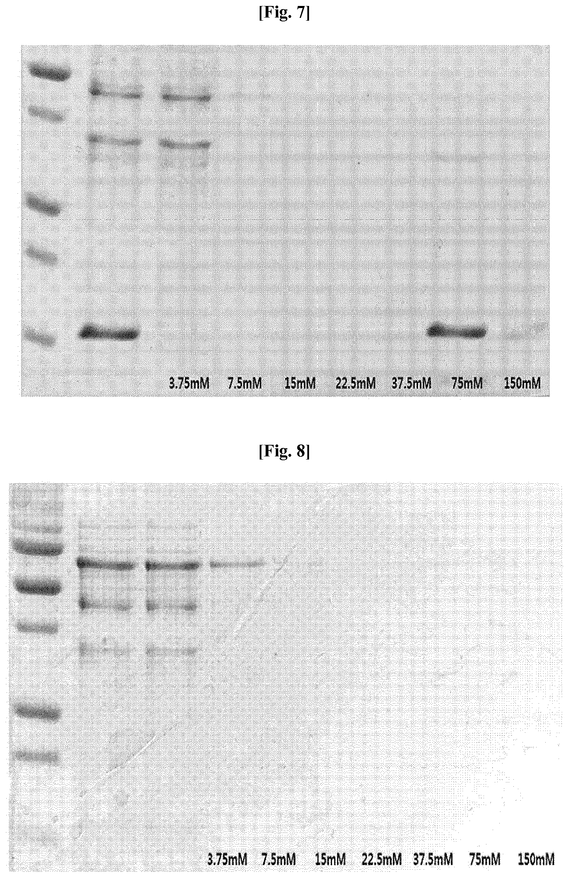

For purification of *beta*-alanyl-CoA:ammonia lyase, the *pETa_his_acl2* plasmid obtained in Example 2-1 was introduced into *E*. *coli* BL21 (DE3) (F- ompT hsdSB(rB- mB-) gal dcm (DE3), a prophage carrying the T7 RNA polymerase gene) (New England Biolabs, USA). *beta*-Alanyl-CoA:ammonia lyase was purified by the method described in Example 1-2. As a result, it was confirmed that the beta-alanyl-CoA:ammonia lyase purified with 75 mM imidazole had the highest purity (Fig. 7). In addition, the buffer was exchanged by flowing a 50 mM potassium phosphate buffer (pH 7.5) using an Amicon Ultra Centrifugal Filter (Merck Millipore, Germany). The purified *beta*-alanyl-CoA:ammonia lyase was mixed with glycerol at a volume ratio of 1:1, and then stored at -80°C.

### Example 3: Confirmation of cloning and activity of propionate coenzyme A transferase

### 3-1: Preparation of pET30a_his_pct vector

PCR was carried out with the primers of SEQ ID NOS: 9 and 10 using chromosomal DNA of *Ralstonia eutropha* as a template. Thereafter, a his-pct fragment encoding propionate coenzyme A transferase with a 6-histidine tag at the N-terminus was prepared.
[SEQ ID NO: 9] pet30a his pct (reh) Fwd: tttaagaaggagatatacatatgcaccatcatcatcatcatAGAAAGGTTCCCATTA TTACC
[SEQ ID NO: 10] pet30a his pct (reh) Rev: gcggccgcaagcttgtcgacTCAGGACTTCATTTCCTTC

A *pET30a_*plasmid capable of expressing a strong gene through the prepared his-pct fragment and a T7 promoter were treated with restriction enzymes (*NdeI* and *SalI*). Thereafter, a recombinant plasmid *pET30a_his_pct* was constructed by ligating the his-pct fragment to the *pET30a_*plasmid through the Gibson Assembly reaction (Fig. 5).

### 3-2: Purification of propionate coenzyme A transferase

For purification of propionate coenzyme A transferase, *the pETa_his_pct* plasmid obtained in Example 3-1 was introduced into *E*. *coli* BL21 (DE3) (F- ompT hsdSB(rB- mB-) gal dcm (DE3), a prophage carrying the T7 RNA polymerase gene) (New England Biolabs, USA). Propionate coenzyme A transferase was purified by the method described in Example 1-2. As a result, it was confirmed that the propionate coenzyme A transferase purified with 3.75 mM imidazole had the highest purity (Fig. 8). In addition, the buffer was exchanged by flowing a 50 mM potassium phosphate buffer (pH 7.5) using an Amicon Ultra Centrifugal Filter (Merck Millipore, Germany). The purified propionate coenzyme A transferase was mixed with glycerol at a volume ratio of 1:1, and then stored at -80°C.

### Example 4: Production of acrylic acid in vitro

Under the conditions described in Table 1 below, a reaction was carried out by the method described in Example 1-3 using the beta-alanine coenzyme A transferase (ACT), beta-alanyl-CoA:ammonia lyase (ACL2), and propionate coenzyme A transferase (PCT) purified in Examples 1-2 and 2-2. In particular, the enzymes to be mixed are mixed at a molar ratio of 1:1:1 (15 µg, 5.5 µg, and 19.5 µg, respectively). In addition, the reaction was carried out at 37°C for 5 hours, and then analyzed by HPLC.

As a result, as shown in Table 1, it was confirmed that acrylic acid was not produced at all when ACT is only mixed with the reaction solution itself, and that acrylic acid (37 mg/L) was produced when ACL2 was additionally added. Further, it was confirmed that the amount of acrylic acid to be produced was increased to 53 mg/L when ACT and ACL2 were added to PCT. It was also confirmed that no acrylic acid was produced when acetyl-CoA was not added to the reaction solution.

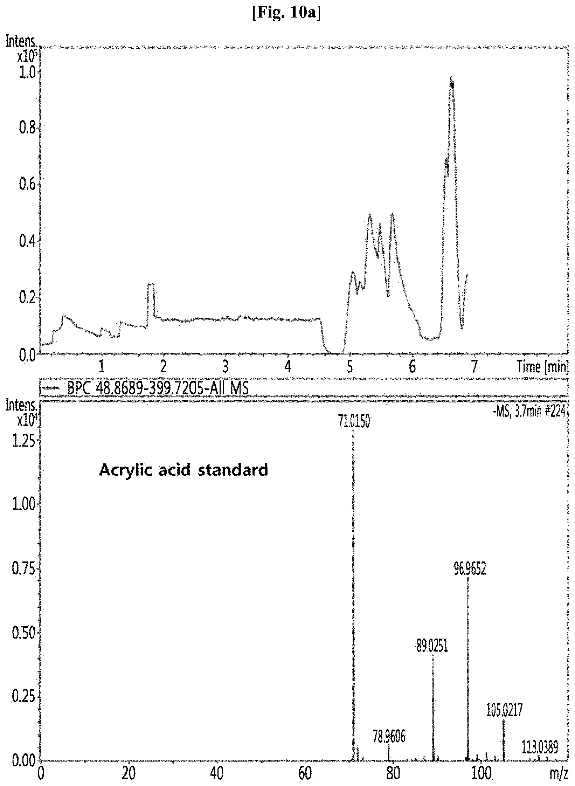

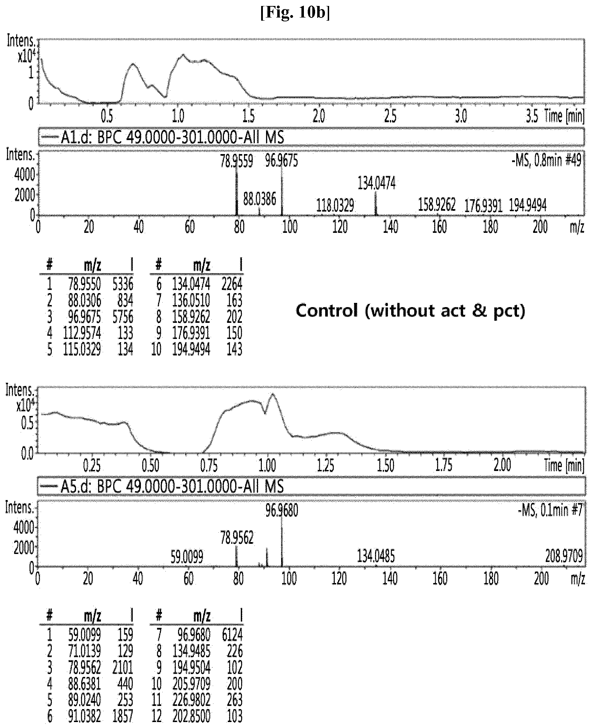

LC MS was used in order to confirm whether the acrylic acid measured by HPLC was actually acrylic acid (Fig. 10).

**[Table 1] Production (mg/L) of acrylic acid according to change of conditions**

| | |
|---|---|
| 10 mM *beta*-alanine + 1 mM acetyl-CoA | 0 |
| 10 mM *beta*-alanine + 1 mM acetyl-CoA + ACT | 0 |
| 10 mM *beta*-alanine + 1 mM acetyl-CoA + ACT + ACL2 | 37 |
| 10 mM *beta*-alanine + 1 mM acetyl-CoA + ACT + ACL2 + PCT | 53 |
| 10 mM *beta*-alanine + ACT + ACL2 + PCT | 0 |

### Example 5: Production of acrylic acid using modified microorganism

### 5-1: Preparation of pTrc99A act, pTrc99A act acl2, and pKYS1 expression vectors

By using chromosomal DNA of the *Clostridium propionicumi* strain and the *Ralstonia eutropha* strain, PCR was carried out with the primers of SEQ ID NOS: 11 and 12 to prepare an *act* fragment encoding *beta*-alanine coenzyme A transferase; PCR was carried out with the primers of SEQ ID NOS: 13 and 14 to prepare an *acl2* fragment encoding *beta*-alanyl-CoA:ammonia lyase; and PCR was carried out with the primers of SEQ ID NOS: 15 and 16 to prepare *a pct* fragment encoding propionate coenzyme A transferase.
[SEQ ID NO: 11] pKYS1aFwd: ctagcgaattcgagctcacaggaaacagaccATGAAAAGACCCTTGGAAG
[SEQ ID NO: 12] pKYS1aRev: gcctgcaggtcgactctagaTTAGATGACATTTTTCTCTTCC
[SEQ ID NO: 13] pKYS1a2Fwd: gagaaaaatgtcatctaatctagaacaggaaacagaccATGGTAGGTAAAAAGGTTGTAC
[SEQ ID NO: 14] pKYS1a2Rev: gcttgcatgcctgcagTTATTGAGGGTGCTTTGC
[SEQ ID NO: 15] pKYS1a2pFwd: caccctcaataactgcagacaggaaacagaccATGAAGGTGATCACCGCACG
[SEQ ID NO: 16] pKYS1a2pRev: ccgccaaaacagccaagcttTTACAGGTGCAGGGGCCC

The prepared *act, acl2,* and *pct* fragments were introduced into a *pTrc99A* plasmid (Pharmacia Biotech, Sweden) capable of expressing a strong gene through a Trc promoter to prepare each of an expression vector expressing *act,* an expression vector expressing *act* and *acl2,* and an expression vector expressing *act, acl2, and pct.*

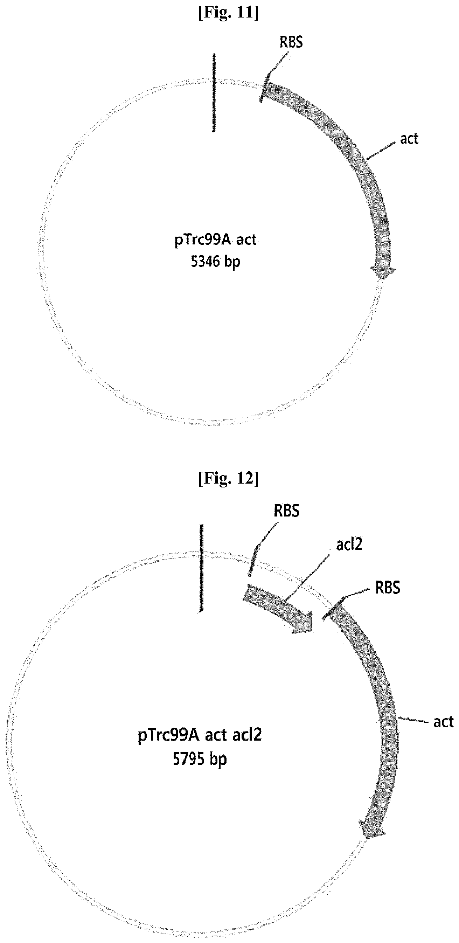

First, *pTrc99A act,* which is vector expressing *act,* was constructed by treating the *pTrc99A* plasmid with restriction enzymes (*SacI* and *kpnI*), followed by ligating the *act* fragment to the *pTrc99A* plasmid through the Gibson Assembly reaction (Fig. 11).

Next, *pTrc99A act acl2,* which is the vector expressing *act* and *acl2,* was constructed by treating the *pTrc99A act* plasmid with restriction enzymes (*SacI* and *xbaI*), followed by ligating the *acl2* fragment to the *pTrc99A act* plasmid through the Gibson Assembly reaction (Fig. 12).

Next, *pKYS1,* which is the vector expressing *act, acl2, and pct,* was constructed by treating the *pTrc99A* plasmid with restriction enzymes (*SacI* and *HindIII*)*,* followed by ligating the *act, acl2, and pct* fragments to the *pTrc99A* plasmid through the Gibson Assembly reaction (Fig. 13).

### 5-2: Production of acrylic acid using modified microorganism

In order to prevent the conversion of acrylyl-CoA to propionyl-CoA, an acrylyl-coA reductase gene in the *CWF4NA pSynPPC7* strain (Song et al., Metab Eng., 30:121-129, 2015), that is a beta-alanine-producing strain, was knocked out, and thereby a *KYS2* strain was prepared.

The *pTrc99A act* plasmid, *pTrc99A act acl2* plasmid, and *pKYS1* plasmid prepared in Example 5-1 were each introduced into the *KYS2* strain. In addition, the strain in which the *pTrc99A* plasmid is introduced into the *KYS2* strain was used as a control strain.

The modified microorganisms into which the respective plasmids prepared in Example 5-1 are introduced were selected on an LB plate medium containing ampicillin (50 mg/L). The transformed strains were inoculated into a 10 mL LB liquid medium containing ampicillin (50 mg/L), and then early incubated for 12 hours with constant shaking at 37°C at 200 rpm. The composition of the modified MR medium (pH 6.5) contained, per liter of triple-distilled water, glucose (15 g), (NH₄)₂SO₄ (9 g), KH₂PO₄ (6.67 g), (NH₄)₂HPO₄ (4.0 g), NaHCO₃ (2 g), citric acid (0.8 g), MgSO₄·7H₂O (0.8 g), CaCl₂·2H₂O (0.01 g), trace metal solution (5 mL) (per liter of distilled water, FeSO₄·7H₂O (10 g), ZnSO₄·4H₂O (2.2 g), MnSO₄·4H₂O (0.58 g), CuSO₄·5H₂O (1 g), (NH₄)₆Mο₇O₂₄·4H₂O (0.1 g), Na₂B₄O₇·10H₂O (0.02 g)). The incubation was carried out in a shaking incubator operated at 37°C at 220 rpm for 36 hours. After the incubation, the culture medium was centrifuged at 10,000 rpm for 10 minutes, and only the supernatant was collected and liquid chromatography analysis was performed, and thereby the production of acrylic acid was measured.

As a result, as shown in Table 2 below, acrylic acid was not produced at all in the strain in which pTrc99A is only introduced and the strain in which only *act* is expressed, whereas 10 mg/L of acrylic acid was produced in the strain int which *act* and *acl2* are introduced. Further, it was confirmed that when *pct* was introduced together with *act* and *acl2,* the amount of acrylic acid to be produced was increased to 15 mg/L.

LC MS was used to confirm whether the acrylic acid measured by HPLC was actually acrylic acid (Table 14).

**[Table 2] Production (mg/L) of acrylic acid according to change of conditions**

| | |
|---|---|
| KYS2 + pTrc99A | 0 |
| KYS2 + pTrc99A *act* | 0 |
| KYS2 + pTrc99A *act acl2* | 10 |
| KYS2 + pTrc99A *act acl2 pct* (pKYS 1) | 15 |

Although the present invention has been described in detail with reference to the specific features, it will be apparent to one of ordinary skill in the art that this description is only for a preferred embodiment and does not limit the scope of the present invention. Thus, the substantial scope of the present invention will be defined by the appended claims

## Claims

1. A modified microorganism producing acrylic acid, wherein the microorganism is a beta-alanine-producing strain, comprising a *act* gene derived from *Clostridium propionicum* encoding a beta-alanine coenzyme A transferase, a *acl2* gene derived from *Clostridium propionicum* encoding a beta-alanyl-CoA:ammonia lyase gene and a pct gene derived from *Ralstonia eutropha* encoding a propionate coenzyme A transferase ; and wherein an *acuI* gene derived from *Escherichia coli* encoding acrylyl-coA reductase is deleted in the microorganism; and wherein the microorganism is *Escherichia* sp.

2. The microorganism according to claim 1, wherein the *act* gene includes the nucleotide sequence of SEQ ID NO: 1.

3. The microorganism according to claim 1, wherein the *acl2* gene includes the nucleotide sequence of SEQ ID NO: 2.

4. The microorganism according to claim 1, wherein the pct gene includes the nucleotide sequence of SEQ ID NO: 3.

5. The microorganism according to claim 1, wherein the *acuI* gene includes the nucleotide sequence of SEQ ID NO: 4.

6. A method for preparing acrylic acid, comprising:
(a) culturing the modified microorganism of any one of claims 1 to 5 in a medium to produce acrylic acid; and
(b) recovering the acrylic acid produced.

7. The method according to claim 6, wherein the medium comprises at least one carbon source selected from the group consisting of glucose, sucrose, lactose, galactose, maltose, xylose, glycerol, fructose, molasses, starch, cellulose, and sugar cane.

8. A method for preparing acrylic acid, comprising:
mixing and reacting beta-alanine coenzyme A transferase encoded by an *act* gene derived from *Clostridium propionicum,* beta-alanyl-CoA:ammonia lyase encoded by an *acl2* gene derived from *Clostridium propionicum* and propionate coenzyme A transferase encoded by a pct gene derived from *Ralstonia eutropha* in a reaction solution comprising beta-alanine.

9. The method according to claim 8, wherein the reaction solution further comprises acetyl coenzyme A.

## Patentansprüche

1. Modifizierter Mikroorganismus, der Acrylsäure produziert, wobei der Mikroorganismus ein Beta-Alanin produzierender Stamm ist, umfassend ein *act*-Gen, das von *Clostridium propionicum* abgeleitet ist und eine Beta-Alanin-Coenzym-A-Transferase codiert, ein *acl2*-Gen, das von *Clostridium propionicum* abgeleitet ist und ein Beta-Alanyl-CoA:Ammoniak-Lyase-Gen codiert, und ein *pct*-Gen, das von *Ralstonia eutropha* abgeleitet ist und eine Propionat-Coenzym-A-Transferase codiert; und wobei ein acul-Gen, das von *Escherichia coli* abgeleitet ist und eine Acrylyl-coA-Reduktase codiert, in dem Mikroorganismus deletiert ist und wobei der Mikroorganismus *Escherichia* sp. ist.

2. Mikroorganismus nach Anspruch 1, wobei das act-Gen die Nukleotidsequenz von SEQ ID NO: 1 beinhaltet.

3. Mikroorganismus nach Anspruch 1, wobei das acl2-Gen die Nukleotidsequenz von SEQ ID NO: 2 beinhaltet.

4. Mikroorganismus nach Anspruch 1, wobei das pct-Gen die Nukleotidsequenz von SEQ ID NO: 3 beinhaltet.

5. Mikroorganismus nach Anspruch 1, wobei das acul-Gen die Nukleotidsequenz von SEQ ID NO: 4 beinhaltet.

6. Verfahren zur Herstellung von Acrylsäure, umfassend:
(a) Kultivieren des modifizierten Mikroorganismus nach einem der Ansprüche 1 bis 5 in einem Medium, um Acrylsäure zu produzieren; und
(b) Gewinnen der produzierten Acrylsäure.

7. Verfahren nach Anspruch 6, wobei das Medium mindestens eine Kohlenstoffquelle umfasst, die aus der Gruppe bestehend aus Glukose, Saccharose, Laktose, Galaktose, Maltose, Xylose, Glycerin, Fruktose, Melasse, Stärke, Cellulose und Zuckerrohr ausgewählt ist.

8. Verfahren zur Herstellung von Acrylsäure, umfassend:
Mischen und Reagieren von Beta-Alanin-Coenzym-A-Transferase, die von einem *act-*Gen codiert wird, das von *Clostridium propionicum* abgeleitet ist, Beta-Alanyl-CoA:Ammoniak-Lyase, die von einem *acl2*-Gen codiert wird, das von *Clostridium propionicum* abgeleitet ist, und Propionat-Coenzym-A-Transferase, die von einem *pct*-Gen codiert wird, das von *Ralstonia eutropha* abgeleitet ist, in einer Reaktionslösung, umfassend Beta-Alanin.

9. Verfahren nach Anspruch 8, wobei die Reaktionslösung weiterhin Acetyl-Coenzym-A umfasst.

## Revendications

1. Microorganisme modifié produisant de l'acide acrylique, lequel microorganisme est une souche productrice de bêta-alanine, comprenant un gène *act* dérivé de *Clostridium propionicum* codant une bêta-alanine coenzyme A transférase, un gène *acl2* dérivé de *Clostridium propionicum* codant un gène de bêta-alanyl-CoA:ammonia lyase et un gène *pct* dérivé de *Ralstonia eutropha* codant une propionate coenzyme A transférase ; et dans lequel un gène *acul* dérivé *d'Escherichia coli* codant l'acrylyl-CoA réductase est supprimé dans le microorganisme ; et lequel microorganisme est *Escherichia* sp.

2. Microorganisme selon la revendication 1, dans lequel le gène *act* contient la séquence de nucléotides de la SEQ ID NO : 1.

3. Microorganisme selon la revendication 1, dans lequel le gène *acl2* contient la séquence de nucléotides de la SEQ ID NO : 2.

4. Microorganisme selon la revendication 1, dans lequel le gène *pct* contient la séquence de nucléotides de la SEQ ID NO : 3.

5. Microorganisme selon la revendication 1, dans lequel le gène *acul* contient la séquence de nucléotides de la SEQ ID NO : 4.

6. Procédé pour préparer de l'acide acrylique, comprenant :
(a) la culture du microorganisme modifié de l'une quelconque des revendications 1 à 5 dans un milieu pour produire de l'acide acrylique ; et
(b) la récupération de l'acide acrylique produit.

7. Procédé selon la revendication 6, dans lequel le milieu comprend au moins une source de carbone choisie dans le groupe constitué par le glucose, le saccharose, le lactose, le galactose, le maltose, le xylose, le glycérol, le fructose, les mélasses, l'amidon, la cellulose, et la canne à sucre.

8. Procédé pour préparer de l'acide acrylique, comprenant :
le mélange et la réaction de bêta-alanine coenzyme A transférase codée par un gène *act* dérivé de *Clostridium propionicum,* de bêta-alanyl-CoA:ammonia lyase codée par un gène *acl2* dérivé de *Clostridium propionicum,* et de propionate coenzyme A transférase codée par un gène *pct* dérivé de *Ralstonia eutropha* dans une solution réactionnelle comprenant de la bêta-alanine.

9. Procédé selon la revendication 8, dans lequel la solution réactionnelle comprend en outre de l'acétyl coenzyme A.
